# EUROPÄISCHE PATENTSCHRIFT

(11) **EP 0 790 859 B1**
(45) Veröffentlichungstag und Bekanntmachung des Hinweises auf die Patenterteilung: **06.05.1998**
(21) Anmeldenummer: 95935859.9
(22) Anmeldetag: 04.11.1995
(51) Int. Cl.: B01J 19/28, B01J 8/10, C07C 29/70, C07C 31/32

(54) **VORRICHTUNG ZUM IN-KONTAKT-BRINGEN VON FESTKÖRPERN IN FORM VON SCHÜTTFÄHIGEN STÜCKEN UND VERFAHREN**
DEVICE FOR BRINGING POURABLE SOLIDS INTO CONTACT WITH GASES OR LIQUIDS
PROCEDE DE MISE EN CONTACT DE CORPS SOLIDES SOUS LA FORME DE MORCEAUX POUVANT ETRE VERSES ET PROCEDE

(30) Priorität: 08.11.1994 DE 4439860
(43) Veröffentlichungstag der Anmeldung: 27.08.1997
(73) Patentinhaber: RWE-DEA AKTIENGESELLSCHAFT FÜR MINERALOEL UND CHEMIE, 22297 Hamburg (DE)
(72) Erfinder: ALBERT, Gert, D-25541 Brunsbüttel (DE)
(74) Vertreter: Schupfner, Gerhard D.
(86) Internationale Anmeldenummer: DE9501552
(87) Internationale Veröffentlichungsnummer: WO9614150

(56) Entgegenhaltungen:
- EP-A- 0 149 151
- CH-A- 439 238
- DE-A- 2 936 023
- DE-A- 4 026 938
- DE-A- 4 336 268
- FR-A- 529 297
- US-A- 2 390 388
- US-A- 3 869 389
- US-A- 5 224 994

## Beschreibung

Die Erfindung bezieht sich auf eine Vorrichtung zum In-Kontakt-Bringen von Festkörpern in Form von schüttfähigen Stücken mit Flüssigkeiten und ggf. Gasen in einem Reaktor durch Zusammenführen der Reaktanden , wobei der Reaktor ein Reaktorgehäuse und im Reaktorgehäuse eine Siebauflage aufweist, in deren Bereich die Kontaktierung erfolgt.

Aus der DE 32 44 972 C1 sind ein Verfahren und eine Vorrichtung zur kontinuierlichen Herstellung von Alumuniumalkoholaten bekannt. In einem Reaktorgehäuse befindet sich dabei ein Siebboden in Korbform, auf den eine Schüttung aus Alumunium in sehr kleiner Partikelgröße, beispielsweise in Nadelform, aufgebracht ist. Der Siebkorb bildet einen Reaktionsraum, der sich oberhalb einer Wanne befindet. Um zu einer ausreichend schnellen Umsetzung zu kommen, müssen die Aluminiumpartikel in dem Reaktionsraum in der Schwebe gehalten werden. Dies erfolgt in der Weise, daß ständig eine sehr große Flüssigkeitsmenge (Alkoholat) über Rohrleitungen in die Wanne und durch den über dieser befindlichen Siebboden des Siebkorbes in den Reaktionsraum gepumpt wird. Durch Löcher in dem Wannenboden fließt ein großer Teil der eingepumpten Flüssigkeit in die Pumpenvorlage ab, bevor sie den Reaktionsraum erreicht. Die Flüssigkeit, die in den Reaktionsraum gelangt ist, fließt aus diesem über die seitlichen Korbwände ab. Das heftige Umwälzen und das Abfließen der Flüssigkeit durch die Löcher in der Wanne zurück in die Pumpenvorlage stellt eine deutliche Energieverschwendung dar. Um die von unten durch den Siebboden eingespeiste Flüssigkeitsmenge zum Fluidisieren in einer ökonomisch realistischen Größen ordnung zu halten, müssen die aufzulösenden Metallpartikel sehr klein sein. Die üblicherweise verwendeten nadelförmigen Metallpartikel sind beispielsweise 5-12 mm lang und haben einen Durchmesser von 0,5- 0,8 mm. Anderenfalls sedimentieren die Metallpartikel sehr schnell und klumpen auf dem Boden des Siebkorbes. Dies führt überwiegend oder total zu einem Blockieren des Siebkorbes. Der Reaktorbetrieb kommt damit zum Erliegen.

Ein weiterer Nachteil des bekannten Reaktors ist darin zu sehen, daß die Herstellung der kleinen, nadelförmigen Metallpartikel sehr teuer ist. Zusammen mit der Notwendigkeit des Umpumpens von riesigen Flüssigkeitsmengen und der Aufteilung in eine obere und eine untere Zugabe, die einen beachtlichen Energie- und Investitionsaufwand bedeuten, ist der Betrieb des bekannten Reaktors ungünstig.

Die Reaktanden lassen sich bei dem bekannten Reaktor im Falle einer Störung, beispielsweise einer Verschmutzung der Siebauflage, nicht schnell und komplett voneinander trennen. Die Beherrschung der exothermen Reaktion von Aluminium mit Alkohol ist somit nicht immer absolut sichergestellt.

Aus der DE 40 26 938 A1 ist ein Drehsäulenreaktor bekannt, der eine gelochte Siebtrommel aufweist. Der Reaktor dient der Gewinnung von Substanzen durch Adsorbtion. Eine Reaktionsbehandlung mit dem Ziel der Auflösung von Festkörpern, insbesondere von Aluminiumstücken mittels Alkohols und deren speziellen Probleme sind dabei nicht angesprochen.

Es ist Aufgabe der Erfindung, eine Vorrichtung der eingangs erwähnten Art zum Auflösen von Festkörpern zu schaffen, die wirtschaftlicher und sicherer arbeitet.

Die gestellte Aufgabe ist erfindungsgemäß dadurch gelöst, daß
- die Siebauflage als rotierende Siebtrommel ausgebildet ist,
- die Siebtrommel während des Betriebes in einer Bodenwanne drehbar ist, in der sich ein mit den Festkörpern reagierendes gasförmiges oder flüssiges Medium befindet,
- die Bodenwanne soweit absenkbar ist, daß die Festkörperstücke in der Siebtrommel und das Medium in der Bodenwanne zur Änderung des Reaktionsverhaltens oder zur Unterbrechung der Reaktion durch das Absenken der Bodenwanne voneinander trennbar sind.

Neben dem Auflösen von Festkörpern können in einer solchen Vorrichtung chemische Umsetzungen oder physikalische Vorgänge wie katalytische Behandlungen, Impfungen, Färbungen, Desinfektion, Adsorption, Wärmebehandlung, Kältebehandlung und/oder Mischen durchgeführt werden.

Bei einer Ausbildung als rotierende Siebtrommel erübrigt sich das volumenmäßig große Umwälzen der Reaktionsflüssigkeit. Die Umwälzung der Feststoffe übernimmt die Siebtrommel, die nun für eine allseitige Berührung mit dem gasförmigen oder flüssigen Medium im Reaktor bzw. in der Wanne sorgt. Die Festkörperstücke brauchen nicht mehr durch Aufwirbelung in der Schwebe gehalten zu werden . Die Festkörperstücke können nun wesentlich größer werden. Es ist vorgesehen, Festkörperstücke in der Form von Pellets, Würfeln, Quadern und auch deutlich größer zum Einsatz zu bringen. Hierunter sind Abschnitte von handelsüblichen Barren oder auch ganze Barren bis zu 25 kg Gesamtgewicht zu verstehen. Solche Festkörperstücke sind deutlich günstiger herstellbar, ganze Barren sogar ohne Extrakosten verfügbar. Zusammen mit der Einsparung eines heftigen Umwälzens des Mediums und den preisgünstigeren Festkörperstücken ergibt sich ein wirtschaflicheres Arbeiten. Die absenkbare Bodenwanne ermöglicht ein rasches Trennen der Reaktanden im Reaktionsraum und zwar ganz nach Wunsch zwischen 0 und 100%. Damit ist gleichzeitig eine feinfühligere Reaktionsführung neben einer deutlichen Erhöhung der Betriebssicherheit gegeben.

Durch den Einsatz der sich drehenden Siebtrommel ergibt sich eine vollständige Durchlässigkeit des Reaktionsraumes der Trommel. Im unteren Bereich des Reaktorgehäuses kann die Flüssigkeit von der Bodenwanne in die Trommel und aus der Trommel in die Bodenwanne strömen. Im oberen Bereich können die Reaktionsgase und -dämpfe aus der Trommel in das Reaktorgehäuse und von dort zur Abkühlung bzw. Kondensation gebracht werden. Bei der Verwendung von Alkohol als Medium ist das Reaktionsprodukt Wasserstoff. Als Medium zur Abfuhr der Wärme aus der stark exothermen Reaktion dient Alkoholdampf. Die Trommel ist in allen Wandbereichen für die aufgegebene Reaktionsflüssigkeit durchlässig.

Der flüssigkeitsfreie Querschnitt in der oberen Hälfte der Siebtrommel soll so groß gewählt werden, daß extrem große Gas- und Dampfmengen abströmen können.

Ein weiterer Vorteil der Vorrichtung ist darin zu sehen, daß das Reaktionsvolumen an den Durchsatz, bzw. die Reaktionsgeschwindigkeit der Festkörperstücke anpaßbar ist durch eine konstruktiv einfache Trommelverlängerung und auch durch eine Höhenverschiebung der Bodenwanne.

Nach einer weiteren Ausgestaltung der Erfindung ist vorgesehen, daß eine in die innere Siebtrommel hineingeführte Lanze vorgesehen ist, mittels des frischen Mediums entweder gleichmäßig über die ganze Trommellänge oder aber in bestimmte Bereiche der Trommel einbringbar ist. Damit wird in der inneren Trommel gezielt eine besonders schnelle Reaktion herbeigeführt.

Nach einer weiteren Ausgestaltung der Erfindung ist vorgesehen, daß zwei Siebtrommeln konzentrisch ineinander angeordnet sind, wobei die Schlitzbreite der Siebe der inneren Siebtrommel größer ist als die Schlitzbreite der Siebe der äußeren Siebtrommel. Damit ergibt sich die Möglichkeit, die Festkörperstücke vorzuwärmen und im Rahmen einer Vorzersetzung zunächst in der inneren Trommel an der Oberfläche abzutragen, bis diese dann durch die Schlitze in der inneren Trommel in die äußere Trommel durchfallen und dort dann vollständig aufgelöst werden. Die innere Trommel dient bei großen Feststoffstücken gleichzeitig als mechanischer Schutz der äußeren feineren Siebe.

Nach einer weiteren Ausgestaltung der Erfindung ist vorgesehen, daß die Festkörperstücke durch die Trommelwelle in das Trommelinnere einführbar sind. Dadurch wird ein kontinuierliches, unproblematisches Laden möglich.

Nach einer weiteren Ausgestaltung der Erfindung ist vorgesehen, daß im Trommelinneren Umwälzschaufeln für die Festkörperstücke vorgesehen sind. Die Umwälzschaufeln fördern bei sich drehender Siebtrommel die Umwälzung der Festkörperstücke horizontal und vertikal und sorgen für eine gleichmäßige Verteilung.

Nach einer weiteren Ausgestaltung der Erfindung ist vorgesehen, daß eine Düsenleiste vorgesehen ist, mittels der das Medium zum Reinigen der Siebtrommelwand auf deren Außenwand aufspritzbar ist. Jeder Siebdrehtrommelbereich bewegt sich beim Drehen der Siebtrommel an den Düsen vorbei. Durch das Aufspritzen mittels Hochdruckstrahlen erfolgt eine optimale Reinigung. Das Aufspritzen reinigt die äußere Trommel kontinuierlich während des Betriebes, so daß es nicht nötig ist, die Vorrichtung zum Reinigen anzuhalten oder sogar auszubauen. Es kann soviel Medium auf die Trommel gespritzt werden, wie zum Nachfüllen oder Nachliefern für den Umsetzprozeß und die Kühlung nötig ist. Die Reinigungs- oder Spülflüssigkeit ist damit zugleich die Reaktions- und Kühlflüssigkeit.

Nach einer weiteren Ausgestaltung der Erfindung ist vorgesehen, daß im Trommelinneren frei bewegliche Mahlkörper vorgesehen sind. Diese Mahlkörper können beispielsweise Kugeln, Würfel oder Zylinder sein, die aus einem Material bestehen, das vom Medium nicht angegriffen wird. Beim mechanischen Umwälzen des festen Trommelinhaltes läuft beim Drehen der Siebtrommel bereits ein Mahlvorgang an den Festkörperstücken ab. Dieser Mahlvorgang und die Auflösungsgeschwindigkeit werden durch die zusätzlichen Mahlkörper vorteilhaft beeinflußt, weil die Oberflächenaktivierung und damit die Reaktionsgeschwindigkeit während des Betriebes gefördert werden.

Nach einer weiteren Ausgestaltung der Erfindung ist vorgesehen, daß zur Absenkung der Bodenwanne eine Seilzughebevorrichtung vogesehen ist. Bei einer Variante dieser Ausgestaltung ist vorgesehen, daß zur Anhebung und Absenkung der Bodenwanne eine hydraulische Hebevorrichtung vorgesehen ist. Die Absenkung der Bodenwanne ist für eine rasche Stillsetzung des Betriebes in einem Störfall von großer Bedeutung. Mit der parziellen Absenkung läßt sich die Reaktion steuern. Ganz gleich, ob das Anheben hydraulisch oder über Seilzüge erfolgt, wichtig ist, daß sich die Bodenwanne im Gefahrenfall ohne Hilfsmittel beim Lösen einer nicht dargestellten Bremse absenken läßt und damit eine rasche Trennung von Medium und Festkörperstücken erfolgt, womit die Reaktion stillgesetzt wird.

Nach einer weiteren Ausgestaltung der Erfindung ist vorgesehen, daß die Bodenwanne durch ein Verschwenken um eine zur Trommelwelle parallele Welle kippend absenkbar ist. In diesem Fall, der konstruktiv besonders interessant ist, brauchen die das Absenken bewirkenden Mittel nur auf einer Seite der Bodenwanne zu wirken.

Nach einer weiteren Ausgestaltung der Erfindung ist vorgesehen, daß das Reaktorgehäuse im Querschnitt aus einer oberen und einer unteren Halbzylinderschale besteht, deren Zylinderschalenränder über Verbindungswände verbunden sind. Dieses Gehäuse ist also in Absenkrichtung der Bodenwanne verlängert. Es kann aber aufgrund des in dem Gehäuse herrschenden Druckes notwendig sein, die Steifigkeit des Gehäuses zu verbessern. In einem solchen Fall ist nach einer weiteren Ausgestaltung der Erfindung vorgesehen, daß das Reaktorgehäuse im Querschnitt eine zylindrische Gestalt aufweist.

Nach einer weiteren Ausgestaltung der Erfindung ist vorgesehen, daß die Siebtrommeln zylindrisch ausgebildet sind. Eine mehreckige Gestaltung ist natürlich ebenfalls denkbar.

Nach einer weiteren Ausgestaltung der Erfindung ist vorgesehen, daß die Reaktanden, Festkörperstücke und gasförmige oder flüssige Medien, in Gegen-, Gleich- oder Kreuzstromführung durch die hohlen Trommelwellenenden in die Siebtrommel einführbar sind.

Nach einer weiteren Ausgestaltung der Erfindung ist vorgesehen,daß die in der Siebtrommel umgewälzten Feststoffe mit der Gasatmosphäre im Reaktor reagieren können.

Nach einer weiteren Ausgestaltung der Erfindung ist vorgesehen, daß zum Herstellen von Metallalkoholaten in der Siebtrommel Festkörper aus Aluminium, Magnesium oder Silicium in reiner Form oder in Mischungen oder Legierungen in Form von schüttfähigen Stücken mit C₁- bis C₁₂-Alkoholen in reiner Form oder in Mischungen zusammenführbar sind. Im speziellen Fall der Herstellung von Aluminiumalkoholaten in der Siebtrommel sind Festkörper aus Aluminium in Form von schüttfähigen Stücken mit C₁- bis C₁₂-Alkohol in reiner Form oder in Mischungen zusammenführbar.

Nach einer weiteren Ausgestaltung der Erfindung ist vorgesehen, daß zur Herstellung von Aluminiumalkoholaten in der Siebtrommel Festkörper aus Aluminium in Form von schüttfähigen Stücken mit aliphatischem C₂- bis C₁₀-Alkohol in reiner Form oder in Mischungen, vorzugsweise C₅- bis C₈- Alkohol in reiner Form oder Mischungen und insbesondere C₆- Alkohol zusammenführbar sind.

Die Erfindung wird anhand der Zeichnung näher erläutert.
Es zeigen:
Fig. 1 eine Vorrichtung zum Herstellen von Metallalkoholaten mit einer die Reaktion zwischen dem Aluminium und dem Alkohol fördernden Siebtrommel,
Fig.2 eine Wannenabsenkung innerhalb des Vorrichtungsgehäuses mittels Seilzügen,
Fig.3 eine Wannenabsenkung innerhalb der Vorrichtung mittels hydraulischer Zylinder
Fig.4 und 5 Schnitte durch die inneren und äußeren Stege der Siebtrommeln mit unterschiedlichen Schlitzbreiten,
Fig.6 bis 8 verschiedene Varianten der Bodenwannenabsenkung,
Fig.9 eine Darstellung der Betriebsanordnung der Vorrichtung nach den Fig.1 bis 8.

Die Vorrichtung nach der Erfindung dient ganz allgemein der Förderung einer Reaktion zwischen Festkörpern, die in Form von Stücken vorliegen, mit einem gasförmigen oder flüssigen Medium, wobei dieses Medium angelegt ist, die Festkörper aufzulösen. Als Materialien der Festkörper kommen vorzugsweise Aluminium, Magnesium oder Silicium in Betracht. Auch andere Festkörpermaterialien sind natürlich einsetzbar. Als gasförmiges oder flüssiges Medium kommen alle Medien in Betracht, die zum Auflösen der Festkörpermaterialien geeignet sind. Bei den flüssigen Medien ist dabei insbesondere an C₁ - C₁₂ geradlinige oder verzweigte Alkohole, C₂ - C₁₂ mehrwertige Alkohole sowie an zyklische und aromatische ein- oder mehrwertige Alkohole gedacht. Im folgenden Ausführungsbeispiel wird lediglich als Beispiel die Herstellung von Aluminiumalkoholaten aus Aluminiumstücke und Alkohol behandelt.

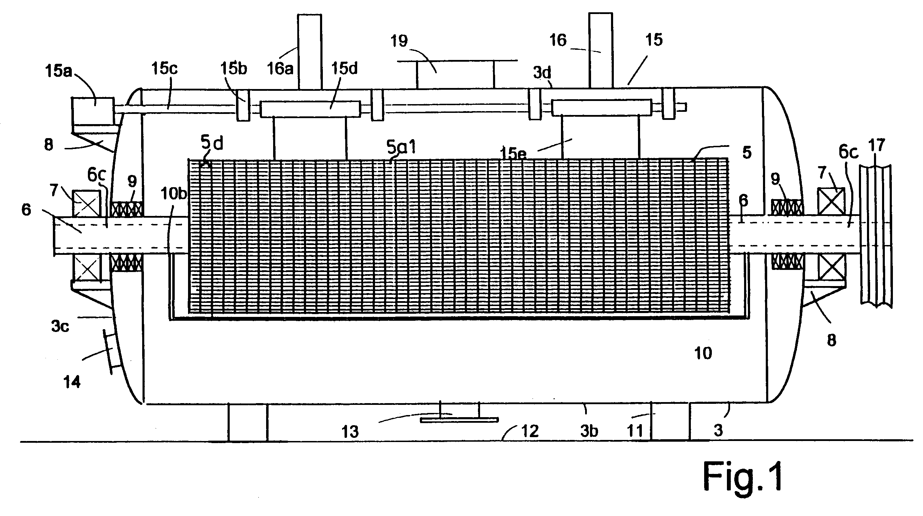

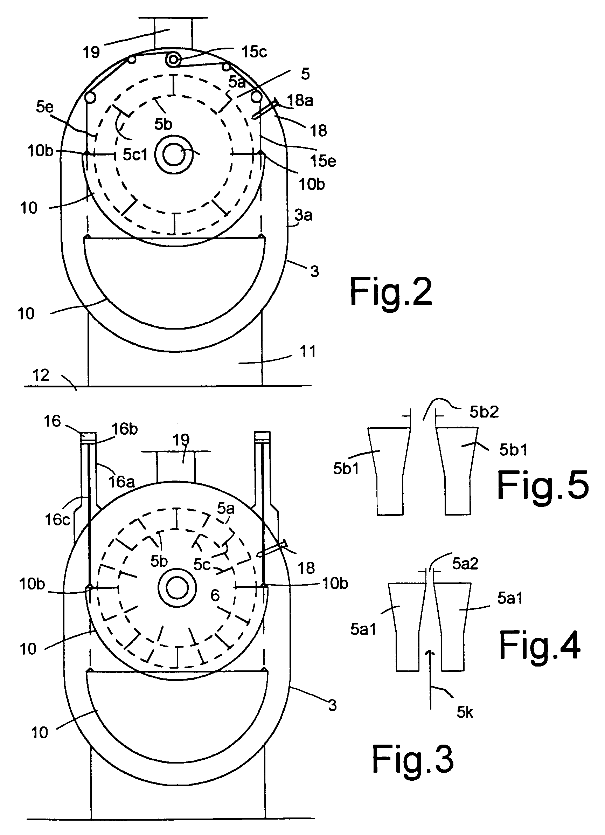

Fig. 1 zeigt eine Vorrichtung zum Herstellen von Aluminiumalkoholaten mit einem Reaktorgehäuse 3, dessen Vorderwand 3a, die aus Fig. 2 zu ersehen ist, in Fig. 1 entfernt ist, um das Innere des Reaktorgehäuses 3 sichtbar zu machen. In dem Reaktorgehäuse 3 befindet sich eine Siebtrommel 5. Diese Siebtrommel 5 ist mit ihrer Welle 6 in Lagern 7 gelagert, die sich am Reaktorgehäuse 3 mittels Stützen 8 abstützen. Die beiden Wellen 6, die durch Dichtungen 9 aus dem Reaktorgehäuse 3 herausführen, sind hohl ausgebildet.

Die untere Hälfte der Siebtrommel 5 befindet sich bei den Darstellungen nach den Fig. 1 und 2 in einer sie aufnehmenden Bodenwanne 10. Diese Bodenwanne 10 ist aus der angehobenen Stellung nach Fig.1 soweit absenkbar, daß sich der in ihr befindliche Alkohol bzw. die aus Alkohol und Aluminium gebildeten Aluminiumalkoholate einerseits und die Aluminiumstücke innerhalb der Siebtrommel 5 andererseits aus dem Reaktionseingriff mit dem Alkohol trennen.

Das Reaktorgehäuse 3 stützt sich mittels Füßen 11 am Boden 12 ab. An der Unterseite 3b des Reaktorgehäuses ist ein Auslaßstutzen 13 für Flüssigkeit vorgesehen. In der linksseitig gesehenen Abschlußwand 3c des Reaktorgehäuses 3 befindet sich ein Mannloch 14.

Zum Absenken und Hochheben der Bodenwanne 10 sind wahlweise eine Seilhebevorrichtung 15 (dargestellt in Fig.2) oder eine hydraulische Hubvorrichtung 16 (dargestellt in Fig.3) vorgesehen. Die seilhebevorrichtung 15 besteht aus einem Motor 15a, der sich außerhalb des Reaktorgehäuses 3 an diesem abstützt. Eine vom Motor 15a antreibbare, in Wellenlagern 15b gelagerte Hubwelle 15c der Seilhebevorrichtung 15 ist an der Oberseite 3d des Reaktorgehäuses 3 im wesentlichen über die Länge des Reaktorgehäuses 3 hinweggeführt. Von Wickelvorrichtungen 15d sind Seile 15e herabgeführt, die, wie Fig. 2 zeigt, an den Oberkanten 10b der Bodenwanne 10 angreifen. Es ist eine nicht dargestellte Seilbremsvorrichtung vorgesehen, mit deren Hilfe die die Bodenwanne 10 haltenden Seile 15e festlegbar sind, um die Bodenwanne 10 in der angehobenen Stellung zu halten.

Fig. 3 zeigt die hydraulische Hubvorrichtung 16 für die Bodenwanne 10. Diese hydraulische Hubvorrichtung besteht aus mehreren Hubzylindern 16a und in diesen angeordneten Hubkolben 16b, die über Hubstangen 16c an den oberen Wannenrändern 10b angreifen.

Die Siebtrommel 5 besteht entweder nur aus einer äußeren Siebtrommel 5a, oder aus zwei konzentrischen Siebtrommeln, nämlich der äußeren Siebtrommel 5a und der inneren Siebtrommel 5b. Die Siebtrommeln 5a und 5b bestehen aus einer nicht näher dargestellten Tragekonstruktion aus Längs - und Querprofilsstegen` die für die Aufnahme von Siebplatten 5a1,5b1 und Umwälzschaufeln 5c ausgebildet sind. Die Siebplatten 5a1 und 5b1 sind über nicht dargestellte Querstege miteinander verbunden, sodaß siebartige Gitter entstehen, die die zu verarbeitende Aluminiumstücke in der Siebtrommel 5 festhalten.. Beide Siebtrommeln 5a und 5b sind über Tragstäbe 5c1 miteinander verbunden. Die Fig. 4 und 5 zeigen Schnitte durch die Längsstege 5a1 und 5b1. Es sind jeweils zwei Längsstege nebeneinander dargestellt. Fig.4 zeigt zwei Längsstege der äußeren Siebtrommel 5a, und Fig.5 zeigt zwei Längsstege der inneren Siebtrommel 5b. Zwischen den Längsstegen 5a1 der äußeren Siebtrommel befinden sich Schlitze 5a2, die sich in der Größenordnung von 0,2 bis 0,4, bevorzugt 0,3 mm bewegen. Zwischen den Längsstegen 5b1 der inneren Siebtrommel 5b sind Schlitze 5b2 in der Größenordnung von 10 mm bis 50 mm vorgesehen. Die Reinigungs-flüssigkeit wird in Richtung von Pfeilen 5k eingespritzt, wodurch die Spalte 5a2 gut gereinigt werden.

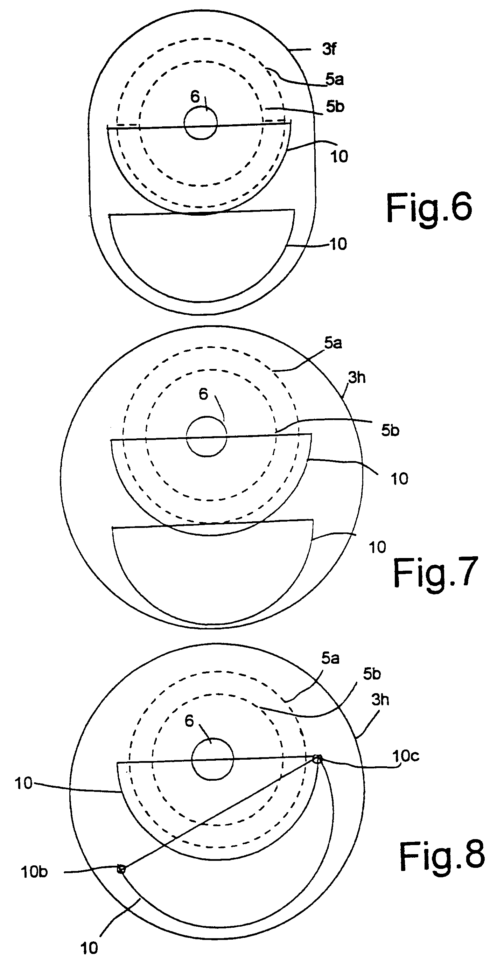

Fig. 6 zeigt ein Reaktorgehäuse 3 mit im Querschnitt und in der Höhe länglicher Form, das aus zwei zylindrischen Halbschalen 3f besteht, die über Verbindungswände 3a miteinander verbunden sind. In diesem in Fig. 6 dargestellten, in der Höhe vergrößerten Reaktorgehäuse 3 läßt sich eine Bodenwanne 10 auch bei großem Siebtrommeldurchmesser vollständig aus dem Wirkbereich der Aluminiumstücke in der Siebtrommel absenken.

Bei höheren Drücken eignet sich besser das in Fig. 7 und 8 dargestellte zylindrische Reaktorgehäuse 3h.

Fig. 8 zeigt eine Ausführungsform wieder mit zylindrischem Reaktorgehäuse 3h. Die Wanne 10 ist aber in diesem Fall nicht wie in den Beispielen nach Fig. 6 und 7 vertikal absenkbar; sie ist vielmehr kippbar um eine Welle 10c, die sich parallel zur Trommelwelle 6 erstreckt. Bei einer derartigen Aufhängung der Wanne 10 werden die Hebevorrichtungen , sei es die Seilhebevorrichtung 15 oder die hydraulische Hubvorrichtung 16, einfacher , weil die Wanne nur an einer Seitenkante 10b gehoben und gesenkt werden muß. Um einen gleichmäßigen Ablauf aus der Wanne zu erhalten, kann die absenkbare Kante mit einem Zackenwehr ausgerüstet werden.

Während des Betriebes wird die Siebtrommel 5 über eine Riemenscheibe 17 in Drehung versetzt. Aluminiumstückchen, die in der Größenordnung von Pellets, Würfel oder Quader oder noch größeren Stücken wie Barrenabschnitte oder ganzen Barren vorliegen können, werden durch die hohle Welle 6 in die Siebtrommel 5 hineingebracht und fallen dabei zunächst in die grobmaschigere Innentrommel 5b. Ebenso können die Reaktanden, die Alumuniumstücke und der Alkohol, in Gegenstromführung durch die beiden hohlen Wellen in die Siebtrommel 5 eingeführt werden. Durch einen Einlaßstutzen 19 wird C₁- C₁₂ - Alkohol in das Reaktorgehäuse eingelassen. In der unteren Hälfte der Siebtrommel, die von der Wanne 10 umschossen ist, befindet sich damit dann ein Gemisch aus Aluminiumstücken und Alkohol. Während des Prozesses, bei dem sich die Siebtrommel 5 dreht, lösen sich die Aluminiumstücke allmählich auf , bis sie so klein geworden sind, daß sie aus der inneren Trommel 5b in die äußere Trommel 5a mit ihrer geringeren Spaltweite durchfallen. Aufgrund der geringeren Spaltbreite der äußeren Trommel 5a besteht die Gefahr, daß sich die Schlitze 5a2 verstopfen. Um dem entgegen zu wirken, ist eine Düsenleiste 18 vorgesehen, die aus mehreren über die Länge der Siebtrommel 5 verteilten Düsen 18a besteht. Über die einzelnen Düsen 18a wird mit hohem Druck Alkohol auf die Außenseite der äußeren Siebtrommelwand 5a aufgespritzt. Damit wird die Siebtrommel 5 während des Betriebes ständig gereinigt.

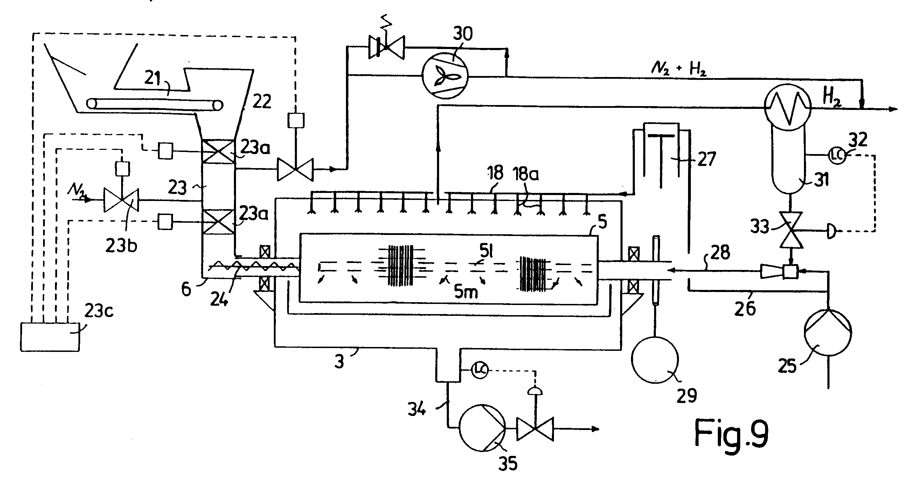

Die Schaltungsanordnung nach Fig. 9 zeigt das Reaktorgehäuse 3 mit der Siebtrommel 5 und der Bodenwanne 10. Zur Dosierung der Aluminiumpartikel dient eine Dosiervorrichtung 21 mit einer Eingabetrichtereinrichtung 22. Aus dieser Eingabetrichtereinrichtung 22 gelangen die Aluminiumpartikel in eine Schleuse 23 mit auf Abstand hintereinandergeschalteten Verschlußventilen 23a, die von einer Schleusensteuerung 23c angesteuert werden. Die Schleusen werden gebraucht, wenn mit Überdruck oder gesundheitsschädlichen oder leicht brennbaren Gasen und Dämpfen gearbeitet wird. Eine Spülung der Schleuse 23 kann z.B. mit Stickstoff oder einem anderen inerten Gas erfolgen mittels eines Ventiles 23b. Die Aluminiumpartikel gelangen stromabwärts der Schleuse 23 zu der hohlen Welle 6 und mittels einer Förderschnecke 24 in die Siebtrommel 5. Die feststehende Schleuse 23 und die rotierende Welle 6 sind über eine Drehdichtung miteinander verbunden. Zum Betrieb wird mittels einer Alkoholpumpe 25 frischer Alkohol in die Vorrichtung eingeführt. Eine Leitung 26 führt zu einer weiteren Pumpe 27, die den Alkohol unter hohem Druck über die Spülleitung 18 und durch die Düsen 18a in die Trommel 5 einspritzt. Für eine Anfangsfüllung oder eine zusätzliche Eingabe frischen Alkohols kann dieser natürlich auch in Richtung eines Pfeiles 28 durch die Welle 6 in die Trommel 5 eingeführt werden, wobei eine Lanze 51 für eine gleichmäßige Verteilung sorgt. Die ist durch kleine Pfeile 5m angedeutet.

Die Trommel 5 wird gedreht von einem Motor 29. Die während des Betriebes entstehenden Wasserstoff- und Alkoholdämpfe strömen über den Stutzen 19 dem Kondensator mit Flüssigkeitessammler 31 zu. Der Alkoholdampf wird kondensiert und anschließend erneut in den Prozeß eingeführt, beispielsweise in Richtung des Pfeiles 28. Die Ableitung aus einer Pumpenvorlage 31 erfolgt höhengesteuert mittels einer Sensors 32 und eines von ihm beaufschlagten Ablaßventiles 33. Der Austrag des Aluminiumalkoholates erfolgt durch eine Leitung 34 mittels einer Alkoholatpumpe 35. Dämpfe und Gase aus der Schleuse 23 werden über das Gebläse 30 abgesaugt und einer Verwertung zugeführt, z.B. dem Wasserstoffstrom unter dem Kondensator zugegeben.

In dem Ausführungsbeispiel wird von einem kontinuierlichen Betrieb ausgegangen. Es ist aber auch ein diskontinuierlicher Betrieb möglich. Dies kann dann erwünscht sein, wenn die Reaktion unterbrochen wird, um einen Teil der Reaktion mit einer Flüssigkeit und einen anderen Teil der Reaktion mit einer anderen Flüssigkeit ablaufen zu lassen. Weiter kann ein Teil der Reaktion in einer Flüssigphase und eine anderer Teil der Reaktion in einer Gasphase ablaufen. Zur Beschleunigung der Reaktion können die Wanne oder/und die Trommel mit Heizrohren versehen werden. Zur Reduzierung der Reaktionsgeschwindigkeit ist auch eine Kühlung, beispielswiese durch Kühlflüssigkeit in den Heizrohren möglich.

Die Reaktionstemperatur läßt sich mittels einer nicht dargestellten, in der Bodenwanne 10 vorgesehenen Meßeinrichtung überwachen.

## Patentansprüche

1. Vorrichtung zum In-Kontakt-Bringen von Festkörpern in Form von schüttfähigen Stücken mit Flüssigkeiten oder von Festkörpern mit Flüssigkeiten und Gasen in einem Reaktor durch Zusammenführen der Reaktanden , wobei der Reaktor ein ReaktorgehäuSe und im Reaktorgehäuse eine Siebauflage aufweist, in deren Bereich die Kontaktierung erfolgt, dadurch gekennzeichnet, daß
- die Siebauflage als rotierende Siebtrommel (5) ausgebildet ist;
- die Siebtrommel (5) während des Betriebes in einer Bodenwanne (10) drehbar ist, in der sich ein mit den Festkörpern reagierendes gasförmiges oder flüssiges Medium befindet; und
- die Bodenwanne (10) soweit absenkbar ist, daß die Festkörperstücke in der Siebtrommel (5) und das Medium in der Bodenwanne (10) zur Änderung des Ausmaßes oder zur Unterbrechung der chemischen Umsetzung oder des physikalischen Vorganges durch das Absenken der Bodenwanne (10) voneinander trennbar sind.

2. Vorrichtung nach Anspruch 1, dadurch gekennzeichnet, daß eine in die innere Siebtrommel (5b) hineingeführte Lanze (5d) vorgesehen ist, mittels der frisches Medium gleichmäßig über die ganze Trommellänge oder in bestimmte Bereiche der Trommel (5) einbringbar ist.

3. Vorrichtung nach Anspruch 1 oder Anspruch 2, dadurch gekennzeichnet, daß zwei Siebtrommeln (5a,5b) konzentrisch ineinander angeordnet sind, wobei die Schlitzbreite der Siebe der inneren Siebtrommel (5b) größer ist als die Schlitzbreite der Siebe der äußeren Siebtrommel (5a).

4. Vorrichtung nach einem der Ansprüche 1-3, dadurch gekennzeichnet, daß die Festkörperstücke durch die Trommelwelle (6) in das Trommelinnere einführbar sind.

5. Vorrichtung nach einem der vorhergehenden Ansprüche, dadurch gekennzeichnet, daß im Trommelinneren Umwälzschaufeln (5c) für die Festkörperstücke vorgesehen sind.

6. Vorrichtung nach einem der vorhergehenden Ansprüche, dadurch gekennzeichnet, daß eine Düsenleiste (18) vorgesehen ist, mittels der das Medium zum Reinigen auf die Außenseite (5e) der Siebtrommel (5a) aufspritzbar ist.

7. Vorrichtung nach einem der vorhergehenden Ansprüche , dadurch gekennzeichnet, daß im Trommelinneren frei bewegliche Mahlkörper vorgesehen sind.

8. Vorrichtung nach einem der vorhergehenden Ansprüche, dadurch gekennzeichnet, daß zur Absenkung der Bodenwanne (10) eine Seilzughebevorrichtung (15) vorgesehen ist.

9. Vorrichtung nach einem der vorhergehenden Ansprüche 1-7, dadurch gekennzeichnet, daß zur Absenkung der Bodenwanne (10) eine hydraulische Hebevorrichtung (16) vorgesehen ist.

10. Vorrichtung nach einem der vorhergehenden Ansprüche, dadurch gekennzeichnet, daß die Bodenwanne (10) durch ein Verschwenken um eine zur Trommelwelle (6) parallele Welle (10c) kippend absenkbar ist.

11. Vorrichtung nach einem der vorhergehenden Ansprüche, dadurch gekennzeichnet, daß das Reaktorgehäuse (3) im Querschnitt aus einer oberen und einer unteren Halbzylinderschale (3f) besteht, deren Zylinderschalenränder über Verbindungswände (3a) verbunden sind.

12. Vorrichtung nach einem der vorhergehenden Ansprüche 1-10, dadurch gekennzeichnet, daß das Reaktorgehäuse (3h) im Querschnitt eine zylindrische Gestalt aufweist.

13. Vorrichtung nach einem der vorhergehenden Ansprüche, dadurch gekennzeichnet, daß die Siebtrommeln (5a,5b) zylindrisch oder polygonal ausgebildet sind.

14. Vorrichtung nach einem der vorhergehenden Ansprüche, dadurch gekennzeichnet, daß die Reaktanden, Festkörperstücke und die Flüssigkeiten ggf. zusammen mit den Gasen, in Gegenstromführung durch die hohlen Wellen (6) in die Siebtrommel (5) einführbar sind.

15. Vorrichtung nach Anspruch 1, dadurch gekennzeichnet, daß die in der Siebtrommel (5) umgewälzten Feststoffe mit der Gasatmosphäre im Reaktor reagieren können.

16. Verfahren zum In-Kontakt-Bringen von Festkörpern in Form von schüttfähigen Stücken mit Flüssigkeiten oder Flüssigkeiten und Gasen in der Vorrichtung nach einem der vorherigen Ansprüche.

17. Verfahren nach Anspruch 16 zum Auflösen von Festkörpern.

18. Verfahren nach Anspruch 16 zur Herstellung von Metallalkoholaten , dadurch gekennzeichnet, daß man Festkörper aus Aluminium, Magnesium oder Silizium in reiner Form oder in Mischungen oder Legierungen in Form von schüttfähigen Stücken mit C1- bis C12-Alkoholen in reiner Form oder in Mischungen in Kontakt bringt.

19. Verfahren nach Anspruch 18 zur Herstellung von Metallalkoholaten , dadurch gekennzeichnet, daß man Festkörper aus Aluminium in Form von schüttfähigen Stücken mit C1- bis C12-Alkoholen in reiner Form oder in Mischungen in Kontakt bringt.

20. Verfahren nach Anspruch 18 zur Herstellung von Aluminiumalkoholaten, dadurch gekennzeichnet, daß man Festkörper aus Aluminium in Form von schüttfähigen Stücken mit C2- bis C10-Alkoholen in reiner Form oder Mischungen, vorzugsweise mit C5- bis C8- Alkoholen in reiner Form oder Mischungen und insbesondere mit C6-Alkohol in Kontakt bringt.

## Claims

1. An apparatus for bringing solids in the form of pourable pieces into contact with fluids or for bringing solids into contact with fluids and gases in a reactor by guiding the reactants together, the reactor comprising a reactor housing with a screen support in the reactor housing and, in this latter, a screening support in the area of which contacting takes place, characterised in that
- the screening support is constructed as a rotating screening drum (5);
- the screening drum (5) is, when in operation, rotatable in a bottom tank (10) in which there is a gaseous or liquid medium which reacts with the solids, and
- the bottom tank (10) can be lowered until such time as the solid pieces in the screening drum (5) and the medium in the bottom tank (10) can be separated from each other in order to vary the degree of or to interrupt the chemical reaction or the physical process by the lowering of the bottom tank (1O).

2. An apparatus according to claim 1, characterised in that there is introduced into the interior screening drum (5b) a lance (5d) by means of which fresh medium can be incorporated regularly over the entire drum length or into specific areas of the drum (5).

3. An apparatus according to claim 1 or claim 2, characterised in that two screening drums (5a, 5b) are disposed concentrically one into the other, the slot width of the screens on the inner screening drum (5b) being greater than the slot width of the screens on the outer screening drum (5a).

4. An apparatus according to one of claims 1 to 3, characterised in that the pieces of solids can be introduced into the interior of the drum through the drum shaft (6).

5. An apparatus according to one of the preceding claims, characterised in that circulating blades (5c) for the pieces of solids are provided in the interior of the drum.

6. An apparatus according to one of the preceding claims, characterised in that a jet bar (18) is provided by means of which the cleaning medium can be sprayed onto the outside (5e) of the screening drum (5a).

7. An apparatus according to one of the preceding claims, characterised in that freely movable grinding members are provided in the drum interior.

8. An apparatus according to one of the preceding claims, characterised in that a cable-operated lifting device (15) is provided for lowering the bottom tank (10).

9. An apparatus according to one of the preceding claims 1 to 7, characterised in that a hydraulic lifting device (16) is provided for lowering the bottom tank (10).

10. An apparatus according to one of the preceding claims, characterised in that the bottom tank (10) can be tiltingly lowered by pivoting about a shaft (10c) parallel with the drum shaft (6).

11. An apparatus according to one of the preceding claims, characterised in that in cross-section the reactor housing (3) consist of an upper and a lower hemi-cylindrical shell (3f) of which the shell edges are connected by connecting walls (3a).

12. An apparatus according to one of the preceding claims 1 to 10, characterised in that the reactor housing (3h) is of cylindrical cross-section..

13. An apparatus according to one of the preceding claims, characterised in that the screening drums (5a, 5b) are of cylindrical or polygonal construction.

14. An apparatus according to one of the preceding claims, characterised in that the reactants, pieces of solids and the liquids can be introduced into the screening drum (5) through the hollow shafts (6) in contra-flow, possibly together with the gases.

15. An apparatus according to claim 1, characterised in that the solids circulated in the screening drum (5) are able to react with the gas atmosphere in the reactor.

16. A method of bringing solids in the form of pourable pieces into contact with fluids or liquids and gases in the apparatus according to one of the preceding claims.

17. A method according to claim 16 for the dissolution of solids.

18. A method according to claim 16 for the production of metal alcoholates, characterised in that solids of aluminium, magnesium or silicon in pure form or in mixtures or alloys in the form of pourable pieces are brought into contact with C1 to C12 alcohols in pure form or in mixtures.

19. A method according to claim 18 for producing metal alcoholates, characterised in that solids of aluminium in the form of pourable pieces are brought into contact with C1 to C12 alcohols in pure form or in mixtures.

20. A method according to claim 18 for producing aluminium alcoholates, characterised in that solids of aluminium in the form of pourable pieces are brought into contact with C2 to C10 alcohols in pure form or mixtures, preferably with C5 to C8 alcohols in pure form or mixtures and in particular with C6 alcohol.

## Revendications

1. Dispositif de mise en contact de corps solides, sous la forme de morceaux aptes à être déversés, avec des liquides ou de corps solides avec des liquides et des gaz dans un réacteur par admission simultanée des réactifs, le réacteur présentant une enveloppe de réacteur et, dans l'enveloppe de réacteur, un montage tamisant, au niveau duquel la mise en contact a lieu, caractérisé en ce que
- le montage tamisant est réalisé sous la forme d'un tambour tamisant rotatif (5);
- le tambour tamisant (5), en service, peut tourner dans une cuve de fond (10) dans laquelle se trouve un milieu gazeux ou liquide réagissant avec les corps solides, et
- la cuve de fond (10) pour abaissée dans une mesure telle que les morceaux de corps solide dans le tambour tamisant (5) et le milieu dans la cuve de fond (10) peuvent être séparés l'un de l'autre par l'abaissement de la cuve de fond (10) pour modifier l'ampleur de la réaction chimique ou du processus physique ou l'interrompre.

2. Dispositif suivant la revendication 1, caractérisé en ce qu'une lance (5d) introduite dans le tambour tamisant intérieur (5b) est prévue, à l'aide de laquelle du milieu frais peut être introduit uniformément sur toute la longueur du tambour ou dans certaines zones du tambour (5).

3. Dispositif suivant la revendication 1 ou la revendication 2, caractérisé en ce que deux tambours tamisants (5a, 5b) sont disposés de manière concentrique l'un dans l'autre, la largeur de fente du tamis du tambour tamisant intérieur (5b) étant supérieure à la largeur de fente du tamis du tambour tamisant extérieur (5a).

4. Dispositif suivant l'une quelconque des revendications 1 à 3, caractérisé en ce que les morceaux de corps solide peuvent être introduits à l'intérieur du tambour à travers l'arbre de tambour (6).

5. Dispositif suivant l'une quelconque des revendications précédentes, caractérisé en ce que, à l'intérieur des tambours, des pales (5c) destinées à remuer les morceaux de corps solide sont prévues.

6. Dispositif suivant l'une quelconque des revendications précédentes, caractérisé en ce qu'une rampe (18) de buses est prévue, à l'aide de laquelle le milieu destiné au nettoyage peut être pulvérisé sur la face extérieure (5e) du tambour tamisant (5a).

7. Dispositif suivant l'une quelconque des revendications précédentes, caractérisé en ce que, a l'intérieur du tambour, des éléments broyeurs à mouvement libre sont prévus.

8. Dispositif suivant l'une quelconque des revendications précédentes, caractérisé en ce que, pour abaisser la cuve de fond (10), un dispositif de levage à câble (15) est prévu.

9. Dispositif suivant l'une quelconque des revendications 1 à 7, caractérisé en ce que, pour abaisser la cuve de fond (10), un dispositif de levage hydraulique (16) est prévu.

10. Dispositif suivant l'une quelconque des revendications précédentes, caractérisé en ce que la cuve de fond (10) peut être abaissée par basculement en la faisant pivoter autour d'un arbre (10c) parallèle à l'arbre de tambour (6).

11. Dispositif suivant l'une quelconque des revendications précédentes, caractérisé en ce que l'enveloppe de réacteur (3) se compose, vue en coupe transversale, d'une coquille semi-cylindrique (3f) supérieure et inférieure dont les bords de coquille cylindrique sont assemblés par des parois d'assemblage (3a).

12. Dispositif suivant l'une quelconque des revendications précédentes, caractérisé en ce que l'enveloppe de réacteur (3h) a, vue en coupe transversale, une forme cylindrique.

13. Dispositif suivant l'une quelconque des revendications précédentes, caractérisé en ce que les tambours tamisants (5a, 5b) présentent une forme cylindrique ou polygonale.

14. Dispositif suivant l'une quelconque des revendications précédentes, caractérisé en ce que les réactifs, les morceaux de corps solide et les liquides peuvent être introduits, facultativement avec les gaz, dans le tambour tamisant (5), à contre-courant, à travers les arbres creux (6).

15. Dispositif suivant la revendication 1, caractérisé en ce que les corps solides remués dans le tambour tamisant (5) peuvent réagir avec l'atmosphère gazeuse dans le réacteur.

16. Procédé de mise en contact de corps solides, sous forme de morceaux aptes à être déversés, avec des liquides ou avec des liquides et des gaz, dans le dispositif suivant l'une quelconque des revendications précédentes.

17. Procédé suivant la revendication 16, destiné à dissoudre les corps solides.

18. Procédé suivant la revendication 16, pour la préparation d'alcoolates de métaux, caractérisé en ce qu'on met en contact des corps solides en aluminium, magnésium ou silicium, à l'état pur ou de mélanges ou d'alliages, sous forme de morceaux aptes à être déversés, avec des alcools en C1 à C12, à l'état pur ou de mélanges.

19. Procédé suivant la revendication 18, pour la préparation d'alcoolates de métaux, caractérisé en ce qu'on met en contact des corps solides en aluminium, sous forme de morceaux aptes à être déversés, avec des alcools en C1 à C12, à l'état pur ou de mélanges.

20. Procédé suivant la revendication 18, pour la préparation d'alcoolates d'aluminium, caractérisé en ce qu'on met en contact des corps solides en aluminium, sous forme de morceaux aptes à être déversés, avec des alcools en C2 à C10, à l'état pur ou de mélanges, de préférence avec des alcools en C5 à C8 à l'état pur ou de mélanges et en particulier, avec de l'alcool en C6.
